# FASCICULE DE BREVET EUROPEEN

(11) **EP 2 861 752 B1**
(45) Date de publication et mention de la délivrance du brevet: **02.12.2020**
(21) Numéro de dépôt: 13733381.1
(22) Date de dépôt: 10.06.2013
(51) Int. Cl.: C12P 21/00, C12N 9/42, C12N 9/24

(54) **PROCÉDÉ DE PRODUCTION D'UN COCKTAIL ENZYMATIQUE UTILISANT LES RÉSIDUS LIQUIDES D'UN PROCÉDÉ DE CONVERSION BIOCHIMIQUE DE MATÉRIAUX LIGNO-CELLULOSIQUES**
VERFAHREN ZUR HERSTELLUNG EINES ENZYMCOCKTAILS UNTER VERWENDUNG DES FLÜSSIGKEITSRESTS AUS EINEM VERFAHREN ZUR BIOCHEMISCHEN UMWANDLUNG VON LIGNOCELLULOSEMATERIALIEN
METHOD FOR PRODUCING AN ENZYME COCKTAIL USING THE LIQUID RESIDUE FROM A METHOD FOR BIOCHEMICALLY CONVERTING LIGNOCELLULOSIC MATERIALS

(30) Priorité: 18.06.2012 FR 1201730
(43) Date de publication de la demande: 22.04.2015
(73) Titulaire: IFP Energies nouvelles, 92500 Rueil-Malmaison (FR); Institut national de recherche pour l'agriculture, l'alimentation et l'environnement, 75007 Paris (FR); Agro Industrie Recherches et Développements, 51110 Pomacle (FR)
(72) Inventeur: BEN CHAABANE, Fadhel, F-75020 Paris (FR); LOURET, Sylvain, F-69003 Lyon (FR)
(74) Mandataire: IFP Energies nouvelles
(86) Numéro de dépôt international: PCT/FR2013/051340
(87) Numéro de publication internationale: WO 2013/190214

(56) Documents cités:
- EP-A1- 2 371 950
- FR-A1- 2 962 444
- FR-A1- 2 981 364
- US-A1- 2010 267 999
- LISA ROSGAARD ET AL: "Comparison of Different Pretreatment Strategies for Enzymatic Hydrolysis of Wheat and Barley Straw", APPLIED BIOCHEMISTRY AND BIOTECHNOLOGY, vol. 143, no. 3, 13 novembre 2007 (2007-11-13), pages 284-296, XP055056020, ISSN: 0273-2289, DOI: 10.1007/s12010-007-8001-6
- WARZYWODA M ET AL: "PRODUCTION AND CHARACTERIZATION OF CELLULOLYTIC ENZYMES FROM TRICHODERMA REESEI GROWN ON VARIOUS CATBON SOURCES", BIORESOURCE TECHNOLOGY, ELSEVIER BV, GB, vol. 39, no. 2, 1 janvier 1992 (1992-01-01), pages 125-130, XP008054567, ISSN: 0960-8524, DOI: 10.1016/0960-8524(92)90130-P
- MIKLÓS GYALAI-KORPOS ET AL: "Cellulase production using different streams of wheat grain- and wheat straw-based ethanol processes", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 38, no. 7, 24 août 2010 (2010-08-24), pages 791-802, XP019919234, ISSN: 1476-5535, DOI: 10.1007/S10295-010-0811-9
- ZSOLT SZENGYEL ET AL: "Cellulose production based on hemicellulose hydrolysate from steam-pretreated willow", APPLIED BIOCHEMISTRY AND BIOTECHNOLOGY, vol. 63-65, no. 1, 1 mars 1997 (1997-03-01), pages 351-362, XP055055714, ISSN: 0273-2289, DOI: 10.1007/BF02920437
- K. RÉCZEY ET AL: "Use of hemicellulose hydrolysate for [beta]-glucosidase fermentation", APPLIED BIOCHEMISTRY AND BIOTECHNOLOGY, vol. 70-72, no. 1, 1 mars 1998 (1998-03-01), pages 225-235, XP055055711, ISSN: 0273-2289, DOI: 10.1007/BF02920139
- MACH R L ET AL: "Regulation of gene expression in industrial fungi: Trichoderma", APPLIED MICROBIOLOGY AND BIOTECHNOLOGY, SPRINGER VERLAG, BERLIN, DE, vol. 60, no. 5, 1 janvier 2003 (2003-01-01), pages 515-522, XP002506248, ISSN: 0175-7598, DOI: 10.1007/S00253-002-1162-X [extrait le 2002-12-03]
- LO C M ET AL: "Cellulase production by continuous culture of Trichoderma reesei Rut C30 using acid hydrolysate prepared to retain more oligosaccharides for induction", BIORESOURCE TECHNOLOGY, ELSEVIER BV, GB, vol. 101, no. 2, 1 janvier 2010 (2010-01-01), pages 717-723, XP026662968, ISSN: 0960-8524, DOI: 10.1016/J.BIORTECH.2009.08.056 [extrait le 2009-09-22]

## Description

### DOMAINE DE L'INVENTION

La présente invention concerne la production des enzymes cellulolytiques et hémicellulolytiques, notamment dans le cadre de la production d'éthanol à partir de matériaux cellulosiques ou ligno-cellulosiques.

### ART ANTÉRIEUR

Depuis les années 1970, la transformation de matériaux ligno-cellulosique en éthanol, après hydrolyse des polysaccharides constitutifs en sucres fermentescibles, a fait l'objet de très nombreux travaux. On peut citer par exemple les travaux de référence du National Renewable Energy Laboratory (Process Design and Economics for Biochemical Conversion of Lignocellulosic Biomass to Ethanol, Humbird et al., NREL/TP-5100-57764, May 2011).

Les matériaux ligno-cellulosiques sont des matériaux cellulosiques, c'est-à-dire constitués à plus de 90% poids de cellulose, et/ou lignocellulosique, c'est-à-dire constitués de cellulose, d'hémicelluloses, qui sont des polysaccharides essentiellement constitués de pentoses et d'hexoses ainsi que de lignine, qui est une macromolécule de structure complexe et de haut poids moléculaire, à base de composés phénoliques.

Le bois, les pailles, les rafles de maïs sont les matériaux ligno-cellulosiques les plus utilisés, mais d'autres ressources, cultures forestières dédiées, résidus de plantes alcooligènes, sucrières et céréalières, produits et résidus de l'industrie papetière et des produits de transformation des matériaux ligno-cellulosiques sont utilisables. Ils sont constitués pour la plupart d'environ 35 à 50 % de cellulose, de 20 à 30 % d'hémicellulose et de 15 à 25 % de lignine.

Le procédé de transformation biochimiques des matériaux ligno-cellulosiques en éthanol comprend une étape de prétraitement physico-chimique, suivie d'une étape d'hydrolyse enzymatique utilisant un cocktail enzymatique, d'une étape de fermentation éthanolique des sucres libérés, la fermentation éthanolique et l'hydrolyse enzymatique pouvant être conduites simultanément, et d'une étape de purification de l'éthanol.

Le cocktail enzymatique est un mélange d'enzymes cellulolytiques (également appelées cellulases) et/ou hémicellulolytiques. Les enzymes cellulolytiques présentent trois grands types d'activités : endoglucanases, exoglucanases et cellobiases, ces dernières étant également appelées β glucosidases. Les enzymes hémicellulolytiques présentent notamment des activités xylanases.

L'hydrolyse enzymatique est efficace et s'effectue dans des conditions douces. En revanche, le coût des enzymes reste très élevé, représentant de 20 à 50% du coût de transformation du matériau ligno-cellulosique en éthanol. De ce fait, beaucoup de travaux ont été conduits pour réduire ce coût : l'optimisation de la production d'enzymes d'abord, en sélectionnant les microorganismes hyperproducteurs et en améliorant les procédés de production desdites enzymes, la diminution de la quantité d'enzymes en hydrolyse ensuite, en optimisant l'étape de prétraitement, en améliorant l'activité spécifique de ces enzymes, et en optimisant la mise en œuvre de l'étape d'hydrolyse enzymatique.

Au cours de la dernière décennie, de nombreux travaux se sont attachés à comprendre les mécanismes d'action et d'expression du cocktail enzymatique. Le but est de faire sécréter le cocktail le plus approprié à l'hydrolyse des matériaux ligno-cellulosiques en modifiant les microorganismes.

Le microorganisme cellulolytique le plus utilisé pour la production industrielle du cocktail enzymatique est le champignon *Trichoderma reesei.* Les souches sauvages ont la faculté de sécréter, en présence d'un substrat carboné inducteur, la cellulose par exemple, le cocktail enzymatique considéré comme le mieux adapté à l'hydrolyse de la cellulose. D'autres protéines possédant des propriétés indispensables à l'hydrolyse des matériaux ligno-cellulosiques sont également produites par *Trichoderma reesei,* les xylanases par exemple. La présence d'un substrat carboné inducteur est indispensable à l'expression des enzymes cellulolytiques et/ou hémicellulolytiques. La nature du substrat carboné a une forte influence sur la composition du cocktail enzymatique. C'est le cas du xylose, qui, associé à un substrat carboné inducteur comme la cellulose ou le lactose, permet d'améliorer significativement l'activité dite xylanase.

Le lactose reste, dans un procédé de production industriel de cocktail enzymatique, un des substrats les plus appropriés; son coût varie cependant de manière importante et représente environ de un à deux tiers du prix de revient des enzymes. Dans le cas de l'utilisation de lactose comme substrat carboné, le procédé de production de cocktail enzymatique est dépendant d'une source de carbone extérieure. De ce fait, l'utilisation de substrats carbonés issus du procédé de conversion biochimique de matériaux ligno-cellulosiques est une voie de progrès importante.

La demande de brevet EP1690944 enseigne que l'extrait de la fraction hémicellulosique sous forme monomère provenant de matériaux ligno-cellulosiques prétraités peut être utilisé comme substrat carboné non inducteur pour la croissance du microorganisme cellulolytique et la production d'enzymes. Dans ce dernier cas, elle doit être mélangée à un substrat carboné inducteur de la production de cellulases (lactose ou cellobiose).

Les demandes de brevet WO09026716 A1 et WO090061486 A1 décrivent la production d'un cocktail enzymatique à partir de *Trichoderma reesei* en utilisant un substrat carboné contenant des sucres inducteurs de la production de cellulases ainsi qu'une source de carbone principale. Cette demande enseigne que 3% poids de sucres inducteurs sont suffisant pour induire la production de cellulases. La production de cellulases est multipliée par un facteur supérieur à 4 par rapport à l'exemple où le xylose est utilisé seul dans la solution de production. Les sucres inducteurs décrits sont des mono-, di- et oligo-saccharides (sucres C₆) produits éventuellement par l'hydrolyse de la cellulose. La source de carbone principale est un ensemble comprenant des mono-, di- et oligo-saccharides issus des hémicelluloses ou du xylose synthétique.

Le document EP 2 371 950 A1 décrit un procédé de production de cellulases basé sur la régulation de l'oscillation de la pression en oxygène dissous dans le milieu de culture. Ce document divulgue que le substrat inducteur carboné est choisi parmi le lactose, le xylose, le cellobiose, le sophorose, des résidus obtenus après fermentation de sucres monomères et/ou un extrait brut de pentoses hydrosolubles, c'est à dire de sucres en C5 hydrosolubles.

La publication "Cellulase Production by Continuous Culture of Trichoderma reesei Rut C30 using acid hydrolysate prepared to retain more oligosaccharides for induction", Lo et al., Bioresource Technology 101 (2010) 717-723, enseigne une induction par les oligosaccharides produites par hydrolyse acide de cellulose, ledit hydrolysat, constitué d'oligomères de sucres en C6, étant basifié par une solution de Ca(OH)2 avant d'être neutralisé. Le rôle inducteur des oligomères C6, en particulier du cellobiose, est connu par ailleurs.+

Le brevet FR 2 962 444 concerne un procédé de production d'enzymes cellulolytiques et/ou hémicellulolytiques par un microorganisme cellulolytique et/ou hémicellulolytique, qui comprend au moins une phase de croissance en présence d'une source de carbone et au moins une phase de production en présence d'un substrat inducteur, dans lequel ledit substrat inducteur est un mélange comprenant de 40 à 65 % poids de glucose ou d'hydrolysats cellulosiques, de 21 à 25 % poids de lactose et de 10 à 39 % poids de xylose ou d'une solution d'hydrolysat hémicellulosique lignocellulosique, la somme de ces trois constituants étant égale à 100 %. Ce document indique que le substrat inducteur est dépourvu de tout autre sucre que les constituants listés ci-dessus, les hydrolysats cellulosiques étant du glucose issu de l'hydrolyse de la cellulose, et les hydrolysats hémicellulosiques étant une solution de sucres en C5.

Un objet de l'invention est de proposer une nouvelle source de carbone inductrice facilement disponible, permettant de produire un cocktail enzymatique aux activités appropriées à l'hydrolyse du matériau ligno-cellulosique. Par rapport au brevet EP1690944, l'invention revendique l'utilisation avantageuse d'une fraction hémicellulosique contenant également des oligomères, ce qui permet de s'affranchir de l'ajout d'un substrat inducteur. Par rapport au brevet WO09026716, l'invention préconise l'utilisation d'une seule source de carbone inductrice non synthétique et particulièrement adaptée à l'expression d'un mélange d'enzymes parfaitement efficace pour le traitement de la biomasse à hydrolyser. Cette invention permet par ailleurs la valorisation en interne de coproduits.

### RÉSUMÉ ET INTÉRÊT DE L'INVENTION

La présente invention concerne un procédé de production d'un cocktail enzymatique par un microorganisme cellulolytique caractérisé en ce qu'il utilise seulement un résidu liquide du prétraitement de matériaux ligno-cellulosiques comme substrat carboné inducteur pour induire la production du cocktail enzymatique.

Un avantage de l'invention est de réduire, voire de supprimer l'apport en substrat carboné d'origine externe au procédé de conversion biochimique de matériaux ligno-cellulosiques. Un autre avantage est de valoriser les résidus liquides dudit procédé de conversion biochimique pour la production d'un cocktail enzymatique. Cette valorisation permet de réduire la quantité d'effluents produits qui doivent être retraités avant rejet ou stockage.

Lesdits résidus liquides contenant des oligomères inducteurs étant valorisés pour la production d'un cocktail enzymatique, le coût dudit cocktail est réduit.

Un avantage supplémentaire du procédé selon l'invention est de produire un cocktail enzymatique particulièrement adapté à l'hydrolyse enzymatique du matériau ligno-cellulosique prétraité converti dans le procédé de conversion biochimique. En particulier, on a découvert un effet positif des oligomères C₅ sur l'activité cellulase des enzymes obtenues.

### DESCRIPTION DÉTAILLÉE DE L'INVENTION

La présente invention est un procédé de production d'un cocktail enzymatique avec un microorganisme cellulolytique comprenant deux phases :
- une phase a) de croissance dudit microorganisme en réacteur fermé en présence d'une solution carbonée de croissance,
- une phase b) de production dudit cocktail enzymatique réalisée avec une alimentation en solution carbonée de production dont la concentration en substrat carboné est comprise entre 150 et 400 g/L, ladite solution carbonée de production comprenant un substrat carboné inducteur,
caractérisé en ce que ledit substrat carboné inducteur est seulement un résidu liquide issu d'une étape de prétraitement de matériaux ligno-cellulosiques, utilisé sans stérilisation, ni modification du pH dudit résidu liquide, dont les oligomères de sucres C₅ représentent au moins 1 % poids des sucres totaux présents dans ledit résidu liquide, et au moins 0,3 % poids des sucres totaux présents dans ladite solution carbonée de production.

Préférentiellement, la solution carbonée de croissance utilisée dans ladite phase a) est à une concentration initiale comprise entre 10 et 90 g de substrat carboné par litre de volume réactionnel.

Préférentiellement, ladite étape de prétraitement est une hydrolyse acide, une cuisson acide ou une explosion à la vapeur avec imprégnation préalable desdits matériaux ligno-cellulosiques avec une solution aqueuse d'acide sulfurique.

Préférentiellement, ledit résidu liquide est utilisé sans stérilisation ni modification du pH dudit résidu liquide. De manière préférée, ledit résidu liquide est utilisé sans stérilisation ni détoxification, ni modification du pH dudit résidu liquide.

Préférentiellement, les oligomères de sucres C₅ représentent entre 1 et 50 % poids des sucres totaux présents dans ledit résidu liquide.

Préférentiellement, ledit substrat carboné inducteur est utilisé seul ou en mélange avec au moins un autre substrat carboné non inducteur.

Préférentiellement, ledit autre substrat carboné non inducteur est choisi parmi le glucose, le xylose, et le saccharose pris seul ou en mélange.

Préférentiellement, ladite solution carbonée de production consiste en un résidu liquide et en au moins un substrat carboné non inducteur, choisi parmi le glucose, le xylose, et le saccharose pris seul ou en mélange, ledit résidu liquide étant issu d'une étape de prétraitement de matériaux ligno-cellulosiques, et utilisé sans stérilisation, ni détoxification, ni modification du pH, ledit résidu liquide consistant en oligomères de sucres C₅, en monomères de sucres C₅ et C₆ et en produits de dégradation des sucres monomères, dont les oligomères de sucres C₅ représentent au moins 1 % poids des sucres totaux présents dans ledit résidu liquide, et au moins 0,3 % poids des sucres totaux présents dans ladite solution carbonée de production.

Préférentiellement, le débit spécifique de l'alimentation en solution carbonée de production de la phase b) est compris entre 35 et 65 mg de substrat carboné par gramme de microorganisme et par heure.

Préférentiellement, le microorganisme cellulolytique est choisi parmi les souches de champignons appartenant aux genres *Trichoderma, Aspergillus, Penicillium* ou *Schizophyllum.*

Préférentiellement, le microorganisme cellulolytique appartient à l'espèce *Trichoderma reesei.*

Ledit procédé de production d'un cocktail enzymatique est mené en culture submergée. Par culture submergée, on entend une culture en milieu liquide.

Les microorganismes cellulolytiques mis en œuvre dans le procédé de production d'un cocktail enzymatique selon l'invention sont des souches de champignons cellulolytiques, par exemple appartenant aux genres *Trichoderma, Aspergillus, Penicillium* ou *Schizophyllum,* de préférence appartenant à l'espèce *Trichoderma reesei.* Les souches industrielles les plus performantes sont les souches appartenant à l'espèce *Trichoderma reesei,* modifiées pour améliorer le cocktail enzymatique par les procédés de mutation-sélection, comme par exemple la souche IFP CL847 (brevet français FR-B-2 555 803). Les souches améliorées par les techniques de recombinaison génétique peuvent être également utilisées. Ces souches sont cultivées en réacteurs agités et aérés dans des conditions compatibles avec leur croissance et la production des enzymes.

Par substrat carboné, on désigne l'ensemble des sucres compris dans la solution carbonée.

La solution carbonée de croissance dudit microorganisme utilisée dans ladite phase a) du procédé selon l'invention est une solution aqueuse comprenant avantageusement un substrat carboné choisi parmi les sucres solubles industriels, et de préférence parmi le glucose, le xylose, les résidus liquides obtenus après fermentation éthanolique des sucres monomères des hydrolysats enzymatiques de matériaux ligno-cellulosiques et les extraits de la fraction hémicellulosique sous forme de monomères provenant de matériaux ligno-cellulosiques prétraités, utilisé seul ou en mélange. Selon sa nature, ladite solution carbonée de croissance est introduite dans le réacteur fermé avant stérilisation ou est stérilisée séparément et introduite dans le réacteur fermé après stérilisation de ce dernier.

De préférence, ladite solution carbonée de croissance est utilisée dans ladite phase a) à une concentration initiale comprise entre 10 et 90 g de substrat carboné par litre de volume réactionnel.

De préférence, ladite phase a) est réalisée sur une durée comprise entre 30 et 70 h, de préférence entre 30 et 40 h.

De préférence, ladite phase a) opère à un pH de 4,8 et à une température de 27°C.

De préférence, ladite phase a) est réalisée dans un réacteur fermé, aéré et agité. L'aération est réglée de manière à obtenir une VVM (débit volumique d'air Nm³/min divisé par le volume réactionnel en m³) comprise entre 0,1 et 1, préférentiellement entre 0,3 et 0,7, et plus préférentiellement de manière à obtenir une VVM de 0,5. L'agitation est adaptée de manière à obtenir une pression partielle en oxygène dissout comprise entre 20 et 80% de la saturation théorique, préférentiellement entre 30 et 50%, et plus préférentiellement à une valeur de 40%.

La solution carbonée de production dudit microorganisme utilisée dans ladite phase b) selon l'invention est une solution aqueuse comprenant un substrat carboné inducteur. Ledit substrat carboné inducteur est un résidu liquide issu de l'étape de prétraitement de matériaux ligno-cellulosiques comprenant des oligomères de sucres C₅.

Conformément à l'invention, lesdits oligomères de sucres C₅ représentent au moins 0,3% poids des sucres totaux contenus dans ladite solution carbonée de production. De préférence, lesdits oligomères de sucres C₅ représentent entre 0,3 et 50% poids des sucres totaux contenus dans ladite solution carbonée de production, de manière préférée entre 0,3 et 20% poids, de manière plus préférée entre 0,3 et 10% poids et de manière encore plus préférée entre 0,3 et 6% poids.

De préférence, ledit substrat carboné inducteur est utilisé seul ou en mélange avec au moins un autre substrat carboné non inducteur.

De préférence, ledit autre substrat carboné non inducteur est choisi parmi des sucres non inducteurs, de manière préférée choisi parmi le glucose, le saccharose et le xylose, pris seul ou en mélange. De manière très préférée, ledit autre substrat carboné est choisi parmi le glucose et le saccharose pris seul ou en mélange.

Conformément à l'invention, ladite solution carbonée de production utilisée dans ladite phase b) selon l'invention est préparée à la concentration de 150 à 400 g de substrat carboné par litre de solution carbonée de production. Le débit spécifique de l'alimentation en solution carbonée de production de ladite phase b) est avantageusement compris entre 35 et 65 mg de substrat carboné par gramme de microorganisme et par heure, préférentiellement de 35 à 45 mg de substrat carboné par gramme de microorganisme et par heure.

De préférence, ladite phase b) est réalisée sur une durée au moins supérieure à 30 h, de préférence au moins supérieure à 100 h.

De préférence, ladite phase b) opère à un pH compris entre 3 et 5,5 et à une température de comprise entre 20 et 30°C.

De préférence, ladite phase b) est réalisée dans un réacteur aéré et agité. L'aération est réglée de manière à obtenir une VVM comprise entre 0,1 et 1, préférentiellement entre 0,3 et 0,7, et plus préférentiellement de manière à obtenir une VVM de 0,5. L'agitation est adaptée de manière à obtenir une pression partielle en oxygène dissout comprise entre 20 et 80%, préférentiellement entre 30 et 50%, et plus préférentiellement à une valeur de 40%.

Ladite phase b) peut être conduite selon les modes fed-batch et chemostat selon la terminologie anglo-saxonne, connus de l'homme du métier.

Les oligomères de sucres C₅ utilisés comme substrat carboné inducteur dans la solution carbonée de production utilisée dans ladite phase b) sont compris dans un résidu liquide issu de l'étape de prétraitement de matériaux ligno-cellulosiques.

Ladite étape de prétraitement du matériau ligno-cellulosique permet d'améliorer la susceptibilité à l'hydrolyse enzymatique de la fraction cellulosique. Ladite étape de prétraitement est une étape de prétraitement physique, comme par exemple une explosion à la vapeur, ou chimique, ou physico-chimique. Dé préférence, ladite étape de prétraitement est une étape de prétraitement acide ou basique, comme par exemple une hydrolyse alcaline, une cuisson alcaline ou une explosion à la vapeur avec imprégnation préalable dudit matériau avec une solution aqueuse alcaline. De préférence, ladite étape de prétraitement est une étape de prétraitement acide, de manière préférée une hydrolyse acide, une cuisson acide ou une explosion à la vapeur avec imprégnation préalable dudit matériau avec une solution aqueuse d'acide sulfurique. De manière très préférée, l'étape de prétraitement est l'explosion à la vapeur.

L'effluent de ladite étape de prétraitement est séparée en deux phases : une phase constituant le résidu solide et une phase constituant le résidu liquide. Ladite séparation peut être effectuée par tout moyen connu de l'homme du métier. Par exemple, ladite séparation peut être effectuée par centrifugation, filtre presse, décantation, ou tout autre moyen technique permettant la séparation d'une phase liquide et d'une phase solide.

Dans le cas d'un prétraitement acide, l'homme du métier ajuste les conditions opératoires (quantité d'acide, taux d'humidité, température, pression, durée) en fonction du matériau ligno-cellulosique à prétraiter et des technologies mises en œuvre. Lesdites conditions opératoires seront plus sévères, par exemple, pour du miscanthus que pour de la paille de blé. Ces ajustements visent à obtenir une hydrolyse complète des hémicelluloses sous forme de monomères, tout en minimisant la formation de produits de dégradation (notamment furfural, 5-HMF). Pour ce faire, l'étape de prétraitement peut également être subdivisée en deux phases : une première phase visant à libérer les oligomères de sucres C₅, suivie d'une phase visant à produire des monomères à partir des oligomères libérés (Process Design and Economics for Biochemical Conversion of Lignocellulosic Biomass to Ethanol, Humbird et al., NREL/TP-5100-57764, May 2011).

Conformément à l'invention, ladite étape de prétraitement est opérée par des moyens connus de l'homme du métier de manière à ce que le résidu liquide comprenne des oligomères de sucres C₅.

Pour obtenir des oligomères de sucres C₅ dans le résidu liquide de ladite étape de prétraitement, on peut abaisser la quantité d'acide mise en œuvre dans ladite étape de prétraitement. On peut également abaisser la température et/ou la pression à laquelle ladite étape de prétraitement est opérée par rapport aux conditions optimisées pour ne libérer que des monomères.

Conformément à l'invention, lesdits oligomères de sucres C₅ compris dans ledit résidu liquide issu de l'étape de prétraitement de matériaux ligno-cellulosiques représentent entre 1 et 100% poids des sucres totaux présents dans ledit résidu liquide, préférentiellement entre 1 et 50% poids, et plus préférentiellement entre 1 et 30% poids.

Ledit résidu liquide contenant les oligomères de sucres C₅ issu de ladite étape de prétraitement est utilisé comme source de carbone inducteur sans qu'il soit nécessaire ni de stériliser ledit résidu liquide, ni de modifier le pH dudit résidu.

Dans un mode de réalisation préféré, le matériau ligno-cellulosique prétraité qui correspond à la fraction dite « solide », est hydrolysé dans une étape d'hydrolyse enzymatique. L'effluent de cette étape est ensuite traité dans une étape de fermentation éthanolique des sucres monomères des hydrolysats enzymatiques. Ces traitements peuvent être réalisés dans le même équipement, ou dans des équipements différents.

Dans un autre mode de réalisation préféré, l'étape de fermentation et au moins une partie de l'étape d'hydrolyse sont réalisées simultanément. Ceci est réalisé, par exemple, en ajoutant des levures éthanoliques au cours de l'étape d'hydrolyse enzymatique.

Les exemples qui suivent illustrent l'invention sans en limiter la portée.

### Exemple 1 (non-conforme) - Production d'un cocktail enzymatique sur glucose

*L'exemple 1 présente une culture utilisant le glucose comme substrat carboné de production. Il s'agit d'un répresseur de la production de cellulases. Cet exemple aboutit à une faible production d'enzymes.*

La production d'un cocktail enzymatique est effectuée en réacteur agité mécaniquement. Le milieu minéral (dit 4N) a la composition suivante : KOH 1,66 g/L, H₃PO₄ 85 % 2 mL/L, (NH₄)₂SO₄ 2,8 g/L, MgSO₄, 7 H₂O 0,6 g/L, CaCL₂ 0,6 g/L, MnSO₄ 3,2 mg/L, ZnSO₄, 7 H₂O 2,8 mg/L, CoCl₂ 10 H₂O 4,0 mg/L, FeSO₄, 7 H₂O 10 mg/L, Corn Steep 1,2 g/L, anti-mousse 0,5 mL/L.

### Préculture liquide

La croissance du microorganisme (la souche de Trichoderma reesei CL847) en préculture est faite en utilisant le glucose comme substrat carboné de croissance, à la concentration de 30 g/L. Le milieu minéral de la préculture est le milieu 4N additionné de phtalate de potassium à 5 g.L⁻¹ afin de tamponner le pH. La croissance de l'inoculum dure 3 jours et est effectuée à 30 °C dans un incubateur agité. Le transfert vers le réacteur est réalisé si la concentration résiduelle en glucose est inférieure à 15 g/L.

### Phase de croissance

Le réacteur contenant le milieu 4N est stérilisé à 120 °C pendant 20 minutes. Le substrat carboné de croissance glucose est stérilisé à part à 120°C pendant 20 minutes puis ajouté stérilement dans le réacteur de façon à avoir une concentration de 30 g/L. Le réacteur est ensemencé à 10% (v/v) avec la préculture liquide de la souche de Trichoderma reesei CL847. Les conditions opératoires sont une température de 27 °C et un pH de 4,8 (régulé par de l'ammoniaque 5.5 mol/L). L'aération est de 0,5 vvm et l'agitation est augmentée entre 200 et 800 rpm en fonction de la pO₂ (pression en oxygène dissous), qui est maintenue à 30 %.

### Phase de production

Lorsque le substrat carboné de croissance du réacteur est épuisé, le substrat carboné de production glucose à 250 g/L est injecté en continu au débit de 35 à 45 mg par g de microorganisme et par heure jusqu'à 164 heures. Les conditions opératoires sont une température de 25 °C et un pH de 4 (régulé par de l'ammoniaque 5.5 mol/L, ce dernier apportant également l'azote nécessaire à la synthèse des protéines excrétées). La teneur en oxygène dissous est maintenue à 30 % par action sur l'agitation.

La production d'enzymes est suivie par le dosage des enzymes extracellulaires par la méthode de Lowry et standard BSA, après séparation du microorganisme par filtration ou centrifugation. Les activités cellulolytiques déterminées sont:
- L'activité papier filtre (UPF : unité papier filtre) qui permet de doser l'activité globale du cocktail enzymatique endoglucanases et exoglucanases
- L' activité aryl β-glucosidase pour les activités spécifiques.

L'activité UPF est mesurée sur papier Whatman n° 1 (Procédure recommandée par la commission biotechnologique IUPAC) à la concentration initiale de 50 g/L ; on détermine la prise d'essai de la solution enzymatique à analyser qui libère l'équivalent de 2 g/L de glucose (dosage colorimétrique) en 60 minutes. Le principe de l'activité papier filtre est de déterminer par dosage au DNS (acide dinitrosalicylique) la quantité de sucres réduits issue d'un papier Whatman N°1 (Procédure recommandée par la commission biotechnologique IUPAC).

Le substrat utilisé pour déterminer l'activité aryl β-glucosidase est le p-nitrophenyl-β-D-glucopyranoside (PNPG). Il est clivé par la β-glucosidase qui libère le p-nitrophenol.

Une unité d'activité aryl β-glucosidase est définie comme la quantité d'enzyme nécessaire pour produire 1 µmol de p-nitrophenol à partir de PNPG par minute et est exprimé en IU/mL.

Les activités spécifiques sont obtenues en divisant les activités exprimées en IU/mL par la concentration en protéines. Elles sont exprimées en IU/mg.

Les déterminations analytiques sur le moût final de l'exemple 1 donnent les résultats suivants :

| | |
|---|---|
| Biomasse g/L | 15,2 |
| Enzymes g/L | 2,9 |
| UPF IU/mL | 1,4 |
| aryl β-Glucosidase spécifique IU/mg | 0,35 |

### Exemple 2 (non-conforme) - Production d'enzymes sur xylose

*L'exemple 2 présente une culture utilisant le xylose comme substrat carboné de production. Il s'agit d'un répresseur de la production de cellulases. Cet exemple aboutit à une faible production d'enzymes.*

La production d'enzymes est menée dans les mêmes conditions que dans l'Exemple 1. Le substrat carboné pendant la phase de croissance est le lactose et pendant la phase de production, le xylose pur.

Après 30 heures de croissance, après l'épuisement du substrat initial, la solution de xylose à 250 g/L est injectée en continu au débit de 35 mg par g de cellules et par heure jusqu'à 164 heures.

Les déterminations analytiques sur le moût final donnent les résultats suivants :

| | |
|---|---|
| Biomasse g/L | 17,3 |
| Enzymes g/L | 3,1 |
| UPF IU/mL | 1,2 |
| aryl β-Glucosidase spécifique IU/mg | 0,1 |

### Exemple 3 (non-conforme) - Production d'enzymes sur lactose

*L'exemple 3 présente une culture utilisant le lactose comme substrat carboné de production. Il s'agit d'un inducteur de la production de cellulases. Cet exemple aboutit à une forte production d'enzymes présentant une bonne activité.*

La production d'enzymes est menée dans les mêmes conditions que dans l'Exemple 1. Le substrat carboné pendant les phases de croissance et de production est le lactose pur. Le lactose est un inducteur important de la production de cellulases. C'est le substrat qui est le plus utilisé industriellement pour la production de cellulases.

Après 30 heures de croissance, après l'épuisement du substrat initial, la solution de fed-batch à 250 g/L est injectée en continu au débit de 35 mg par g de cellules et par heure jusqu'à 164 heures.

Les déterminations analytiques sur le moût final donnent les résultats suivants :

| | |
|---|---|
| Biomasse g/L | 13,5 |
| Enzymes g/L | 37,8 |
| UPF IU/mL | 22,1 |
| aryl β-Glucosidase spécifique IU/mg | 0,96 |

### Exemple 4 (conforme) - Production sur 100% de résidu liquide contenant des oligomères de sucres C₅

*L'exemple 4 présente une culture utilisant le résidu liquide comme substrat carboné de production. Cet exemple aboutit à une production d'enzymes et une activité supérieures à celles obtenues dans les exemples 1 et 2. On montre donc que le résidu liquide utilisé seul induit la production de cellulases. On aboutit à un effet similaire à ce qui est décrit dans le brevet* WO09026716 A1 *mais sans ajout de solution inductrice autre que le résidu liquide.*

La production d'enzymes est menée dans les mêmes conditions que dans l'Exemple 1. Le substrat carboné pendant les phases de croissance est le glucose. Le substrat carboné durant la phase de production est la fraction liquide obtenue après prétraitement appelée « un résidu liquide ». Celui-ci est issu d'un miscanthus prétraitée par explosion à la vapeur à 14.5 bar pendant 2 minutes après imprégnation à 0,65 % de H₂SO₄ puis séparation phase liquide/phase solide.

Sa composition est la suivante:

| | |
|---|---|
| Oligomères | 0,78 g/L |
| Glucose | 8,98 g/L |
| Xylose | 31,44 g/L |
| Galactose | 1,73 g/L |
| Arabinose | 3,85 g/L |
| Acide acétique | 4,14 g/L |
| HMF | 0,14 g/L |
| Furfural | 0,85 g/L |
| **Total** | **51,91 g/L** |

Il est concentré à 300 g/L. Après 30 heures de croissance, après l'épuisement du substrat initial, la solution concentrée d'hydrolysat hémicellulosique est injectée en continu au débit de 35 mg par g de cellules et par heure.

Les déterminations analytiques sur le moût final donnent les résultats suivants :

| | |
|---|---|
| Biomasse g/L | 26,0 |
| Enzymes g/L | 19,2 |
| UPF IU/mL | 4,9 |
| aryl β-Glucosidase spécifique IU/mg | 0,48 |

### Exemple 5 (conforme) - Production sur deux résidus liquides différents comprenant des proportions d'oligomères différentes

*L'exemple 5 présente deux cultures utilisant chacune un résidu liquide comme substrat carboné de production. Cet exemple abouti à une production d'enzymes et une activité supérieures à celles obtenues dans les exemples 1 et 2. On montre que la teneur en oligomères de sucres C₅ a un effet positif sur la quantité et l'activité des enzymes obtenues.*

On produit deux résidus liquides distincts à partir de paille de blé explosée à la vapeur. Les conditions opératoires de l'explosion à la vapeur sont 14,5 bar, 2 minutes. Le résidu liquide C1 est obtenu à partir d'une paille de blé préalablement imprégnée par H₂SO₄ à 0,64%. Le résidu liquide C2 est obtenu à partir d'une paille de blé préalablement imprégnée par H₂SO₄ à 0,32%.

Le résidu liquide C2 contient une proportion plus important d'oligomères issus de l'hydrolyse des hémicelluloses de la paille par rapport à l'hydrolysat C1, du fait de la moindre quantité d'acide utilisée pour l'imprégnation de la paille de blé.

La composition des résidus liquides C1 et C2 est détaillée dans le tableau suivant:

| | **C1** | **C2** |
|---|---|---|
| **monomères** | 50,9 g/L | 33,6 g/L |
| **oligomères** | 0,9 g/L | 14,1 g/L |
| **sucres dégradés** | 2,9 g/L | 1,8 g/L |
| **TOTAL** | 54,7 g/L | 49,5 g/L |

Les monomères correspondent à la somme du glucose, xylose, arabinose, mannose, galactose, rahmnose. Les oligomères correspondent à la somme des oligomères des sucres C₅ (exemple : xylobiose, xylotriose, xyloarabinose). Les sucres dégradés correspondent à la somme de furfural, de 5 HMF, d'acide lévullinique et d'acide formique.

Deux solutions de fed-batchs sont préparées en dissolvant du glucose dans les hydrolysats C1 et C2 de manière à atteindre une concentration totale en sucres de 250 g/L. Le glucose étant un répresseur de la production de cellulases.

Les expériences sont menées dans les mêmes conditions que l'exemple 1.
Les déterminations analytiques sur le moût final donnent les résultats suivants :

| | **Fed-batch C1** | **Fed-batch C2** |
|---|---|---|
| Biomasse g/L | 24,8 | 19,2 |
| Enzymes g/L | 20,9 | 32,9 |
| UPF IU/mL | 11,4 | 15,6 |
| aryl β-Glucosidase IU/mL | 45,2 | 65,1 |

La solution de Fed-batch C2 qui contient plus d'oligomères issus de la fraction hémicellulose aboutit à une production de protéines supérieure à celle de la solution de Fed-batch C1 avec de meilleures activités enzymatiques. Dans cet exemple, la production d'enzymes est réalisée avec des sources de carbone potentiellement issues uniquement du procédé de production d'éthanol à partir de biomasse lignocellulosique, le glucose pouvant être remplacé par un hydrolysat cellulosique.

### Exemple 6 (non conforme) - Production sur résidu liquide (hydrolysat hémicellulosique) ne contenant pas d'oligomères de sucres C₅

*L'exemple 6 montre, en partant du résidu liquide C2 de l'exemple 5, que ce sont bien les oligomères de sucres C₅ qui induisent la production d'enzymes.*

La solution C2 de l'exemple 5 subit au préalable une hydrolyse acide de manière à hydrolyser les oligomères en monomères. La composition de la nouvelle solution C3 est la suivante:

| | **C3** |
|---|---|
| **monomères** | 45,7 g/L |
| **oligomères** | 0,0 g/L |
| **sucres dégradés** | 3,5 g/L |
| **TOTAL** | 49,2 g/L |

La culture est ensuite menée dans les mêmes conditions que l'exemple 5. Une solution de fed-batch est préparée en dissolvant du glucose dans C3 de manière à atteindre une concentration totale en sucres de 250 g/L. Les déterminations analytiques sur le moût final donnent les résultats suivants :

| | |
|---|---|
| Biomasse g/L | 21,2 |
| Enzymes g/L | 3,9 |
| UPF IU/mL | 1,1 |
| aryl β-Glucosidase IU/mg | 0,2 |

Cet exemple montre qu'en l'absence d'oligomères hémicellulosiques, les résidus liquides n'induisent pas la production de cellulases.

## Revendications

1. Procédé de production d'un cocktail enzymatique avec un microorganisme cellulolytique comprenant deux phases :
- une phase a) de croissance dudit microorganisme en réacteur fermé en présence d'une solution carbonée de croissance,
- une phase b) de production dudit cocktail enzymatique réalisée avec une alimentation en solution carbonée de production dont la concentration en substrat carboné est comprise entre 150 et 400 g/L, ladite solution carbonée de production comprenant un substrat carboné inducteur,
**caractérisé en ce que** ledit substrat carboné inducteur est seulement un résidu liquide issu d'une étape de prétraitement de matériaux ligno-cellulosiques, utilisé sans stérilisation, ni modification du pH dudit résidu liquide, dont les oligomères de sucres C₅ représentent au moins 1 % poids des sucres totaux présents dans ledit résidu liquide, et au moins 0,3 % poids des sucres totaux présents dans ladite solution carbonée de production.

2. Procédé selon la revendication 1, dans lequel ledit résidu liquide comprend lesdits oligomères de sucres C₅, des monomères de sucres C5 et C6 et des produits de dégradation des sucres monomères.

3. Procédé selon l'une des revendications précédentes, dans lequel la solution carbonée de croissance utilisée dans ladite phase a) est à une concentration initiale comprise entre 10 et 90 g de substrat carboné par litre de volume réactionnel.

4. Procédé selon l'une des revendications précédentes, dans lequel ladite étape de prétraitement est une hydrolyse acide, une cuisson acide ou une explosion à la vapeur avec imprégnation préalable desdits matériaux ligno-cellulosiques avec une solution aqueuse d'acide sulfurique.

5. Procédé selon l'une des revendications précédentes, dans lequel les oligomères de sucres C₅ représentent entre 0,3 et 20 % poids, de préférence entre 0,3 et 10% poids, des sucres totaux présents dans ladite solution carbonée de production

6. Procédé selon l'une des revendications précédentes dans lequel les oligomères de sucres C₅ représentent entre 1 et 50 % poids, de préférence entre 1 et 30% poids, des sucres totaux présents dans ledit résidu liquide.

7. Procédé selon l'une des revendications précédentes, dans lequel ledit substrat carboné inducteur est utilisé seul ou en mélange avec au moins un autre substrat carboné non inducteur.

8. Procédé selon la revendication précédente dans lequel ledit autre substrat carboné non inducteur est choisi parmi le glucose, le xylose, et le saccharose pris seul ou en mélange.

9. Procédé selon l'une des revendications 1 à 4 dans lequel ladite solution carbonée de production consiste en un résidu liquide et en au moins un substrat carboné non inducteur choisi parmi le glucose, le xylose, et le saccharose pris seul ou en mélange, ledit résidu liquide étant issu d'une étape de prétraitement de matériaux ligno-cellulosiques, et utilisé sans stérilisation, ni modification du pH, ledit résidu liquide consistant en oligomères de sucres C5, en monomères de sucres C5 et C6 et en produits de dégradation des sucres monomères, dont les oligomères de sucres C5 représentent au moins 1 % poids des sucres totaux présents dans ledit résidu liquide, et au moins 0,3 % poids des sucres totaux présents dans ladite solution carbonée de production.

10. Procédé selon l'une des revendications précédentes dans lequel le débit spécifique de l'alimentation en solution carbonée de production de la phase b) est compris entre 35 et 65 mg de substrat carboné par gramme de microorganisme et par heure.

11. Procédé selon l'une des revendications précédentes dans lequel le microorganisme cellulolytique est choisi parmi les souches de champignons appartenant aux genres *Trichoderma, Aspergillus, Penicillium* ou *Schizophyllum.*

12. Procédé selon la revendication précédente dans lequel le microorganisme cellulolytique appartient à l'espèce *Trichoderma reesei.*

## Patentansprüche

1. Verfahren zur Herstellung eines Enzymcocktails mit einem cellulolytischen Mikroorganismus, umfassend zwei Phasen:
- eine Phase a) des Wachstums des Mikroorganismus im geschlossenen Reaktor in Gegenwart einer kohlenstoffhaltigen Wachstumslösung,
- eine Phase b) der Herstellung des Enzymcocktails, die mit einer Beschickung mit kohlenstoffhaltiger Produktionslösung durchgeführt wird, deren Konzentration an kohlenstoffhaltigem Substrat zwischen 150 und 400 g/l beträgt, wobei die kohlenstoffhaltige Produktionslösung ein kohlenstoffhaltiges Induktorsubstrat umfasst,
**dadurch gekennzeichnet, dass** das kohlenstoffhaltige Induktorsubstrat lediglich ein flüssiger Rückstand ist, der aus einem Schritt der Vorbehandlung von Lignocellulose-Rohstoffen stammt und ohne Sterilisation oder Modifikation des pH-Werts des flüssigen Rückstands verwendet wird, dessen C₅-Zuckeroligomere mindestens 1 Gew.-% der in dem flüssigen Rückstand vorhandenen Gesamtzucker sowie mindestens 0,3 Gew.-% der in der kohlenstoffhaltigen Produktionslösung vorhandenen Gesamtzucker ausmachen.

2. Verfahren nach Anspruch 1, wobei der flüssige Rückstand die C₅-Zuckeroligomere, C5- und C6-Zuckermonomere und Abbauprodukte der Zuckermonomere umfasst.

3. Verfahren nach einem der vorhergehenden Ansprüche, wobei die in Phase a) verwendete kohlenstoffhaltige Wachstumslösung eine Anfangskonzentration zwischen 10 und 90 g an kohlenstoffhaltigem Substrat pro Liter Reaktionsvolumen aufweist.

4. Verfahren nach einem der vorhergehenden Ansprüche, wobei es sich bei dem Vorbehandlungsschritt um Säurehydrolyse, Säureaufschluss oder Dampfexplosion mit vorheriger Imprägnierung der Lignocellulose-Rohstoffe mit einer wässrigen Schwefelsäurelösung handelt.

5. Verfahren nach einem der vorhergehenden Ansprüche, wobei die C₅-Zuckeroligomere zwischen 0,3 und 20 Gew.-%, vorzugsweise zwischen 0,3 und 10 Gew.-%, der in der kohlenstoffhaltigen Produktionslösung vorhandenen Gesamtzucker ausmachen.

6. Verfahren nach einem der vorhergehenden Ansprüche, wobei die C₅-Zuckeroligomere zwischen 1 und 50 Gew.-%, vorzugsweise zwischen 1 und 30 Gew.-%, der in dem flüssigen Rückstand vorhandenen Gesamtzucker ausmachen.

7. Verfahren nach einem der vorhergehenden Ansprüche, wobei das kohlenstoffhaltige Induktorsubstrat allein oder im Gemisch mit mindestens einem anderen kohlenstoffhaltigen Nicht-Induktor-Substrat verwendet wird.

8. Verfahren nach dem vorhergehenden Anspruch, wobei das andere kohlenstoffhaltige Nicht-Induktor-Substrat aus Glucose, Xylose oder Saccharose allein oder im Gemisch ausgewählt ist.

9. Verfahren nach einem der Ansprüche 1 bis 4, wobei die kohlenstoffhaltige Produktionslösung aus einem flüssigen Rückstand und aus mindestens einem kohlenstoffhaltigen Nicht-Induktor-Substrat, ausgewählt aus Glucose, Xylose oder Saccharose allein oder im Gemisch, besteht, wobei der flüssige Rückstand aus einem Schritt der Vorbehandlung von Lignocellulose-Rohstoffen stammt und ohne Sterilisation oder Modifikation des pH-Werts verwendet wird, wobei der flüssige Rückstand aus C5-Zuckeroligomeren, aus C5- und C6-Zuckermonomeren und aus Abbauprodukten der Zuckermonomere besteht, worin die C₅-Zuckeroligomere mindestens 1 Gew.-% der in dem flüssigen Rückstand vorhandenen Gesamtzucker sowie mindestens 0,3 Gew.-% der in der kohlenstoffhaltigen Produktionslösung vorhandenen Gesamtzucker ausmachen.

10. Verfahren nach einem der vorhergehenden Ansprüche, wobei der spezifische Beschickungsdurchsatz mit kohlenstoffhaltiger Produktionslösung der Phase b) zwischen 35 und 65 mg kohlenstoffhaltiges Substrat pro Gramm Mikroorganismus und pro Stunde beträgt.

11. Verfahren nach einem der vorhergehenden Ansprüche, wobei der cellulolytische Mikroorganismus aus zu den Gattungen *Trichoderma, Aspergillus, Penicillium* oder *Schizophyllum* gehörenden Pilzstämmen ausgewählt ist.

12. Verfahren nach dem vorhergehenden Anspruch, wobei der cellulolytische Mikroorganismus zur Spezies *Trichoderma reesei* gehört.

## Claims

1. Process for producing an enzymatic cocktail with a cellulolytic microorganism, comprising two phases:
- phase a) of growing said microorganism in a closed reactor in the presence of a carbon-based growth solution,
- a phase b) of producing said enzymatic cocktail, which is carried out with a feed of carbon-based production solution in which the concentration of carbon-based substrate is between 150 and 400 g/l, said carbon-based production solution comprising a carbon-based inducer substrate,
**characterized in that** said carbon-based inducer substrate is solely a liquid residue from a step of pretreatment of lignocellulosic materials, and is used without sterilization or modification of the pH of said liquid residue, in which the oligomers of C₅ sugars represent at least 1 wt% of the total sugars present in said liquid residue, and at least 0.3 wt% of the total sugars present in said carbon-based production solution.

2. Process according to Claim 1, wherein said liquid residue comprises said oligomers of C₅ sugars, monomers of C5 and C6 sugars, and degradation products of the monomeric sugars.

3. Process according to either of the preceding claims, wherein the carbon-based growth solution used in said phase a) is at an initial concentration of between 10 and 90 g of carbon-based substrate per litre of reaction volume.

4. Process according to any of the preceding claims, wherein said pretreatment step is an acidic hydrolysis, an acidic cooking or a steam explosion with prior impregnation of said lignocellulosic materials with an aqueous sulfuric acid solution.

5. Process according to any of the preceding claims, wherein the oligomers of C₅ sugars represent between 0.3 and 20 wt%, preferably between 0.3 and 10 wt%, of the total sugars present in said carbon-based production solution.

6. Process according to any of the preceding claims, wherein the oligomers of C₅ sugars represent between 1 and 50 wt%, preferably between 1 and 30 wt%, of the total sugars present in said liquid residue.

7. Process according to any of the preceding claims, wherein said carbon-based inducer substrate is used alone or in a mixture with at least one other, non-inducer carbon-based substrate.

8. Process according to the preceding claim, wherein said other, non-inducer carbon-based substrate is selected from glucose, xylose and sucrose, alone or in a mixture.

9. Process according to any of Claims 1 to 4, wherein said carbon-based production solution consists of a liquid residue and of at least one non-inducer carbon-based substrate selected from glucose, xylose and sucrose, alone or in a mixture, said liquid residue being obtained from a step of pretreating lignocellulosic materials and being used without sterilization or modification of the pH, said liquid residue consisting of oligomers of C5 sugars, of monomers of C5 and C6 sugars and of degradation products of the monomeric sugars, in which the oligomers of C5 sugars represent at least 1 wt% of the total sugars present in said liquid residue, and at least 0.3 wt% of the total sugars present in said carbon-based production solution.

10. Process according to any of the preceding claims, wherein the specific throughput of the feed of carbon-based production solution of phase b) is between 35 and 65 mg of carbon-based substrate per gram of microorganism per hour.

11. Process according to any of the preceding claims, wherein the cellulolytic microorganism is selected from the fungal strains belonging to the genera *Trichoderma, Aspergillus, Penicillium* or *Schizophyllum.*

12. Process according to the preceding claim, wherein the cellulolytic microorganism belongs to the species *Trichoderma reesei.*
